# EUROPEAN PATENT APPLICATION

(11) **EP 1 359 224 A1**
(43) Date of publication of application: **05.11.2003**
(21) Application number: 02076752.1
(22) Date of filing: 01.05.2002
(51) Int. Cl.: C12P 7/64, C12N 1/12

(54) **A process for production of polyunsaturated fatty acids by marine microorganisms**

(71) Applicant: ATO B.V., 6700 AA Wageningen (NL)
(72) Inventor: De Swaaf, Martin Egbert, 3583 VD Utrecht (NL); Sijtsma, Lolke, 6866 DM Heelsum (NL); Pronk, Jacobus Thomas, 2636 DD Schipluiden (NL)
(74) Representative: van Westenbrugge, Andries

(57) **Abstract**

The present invention relates to a new process of producing polyunsaturated fatty acids by culturing marine microorganisms on a monoalcohol. The new process is particularly suitable for producing docosahexaenoic acid by dinoflagellates (Crypthecodinium cohnii). The process can be performed as a batch, fed-batch, or continuous process.

## Description

### Field of the invention

The present invention relates to a process of producing polyunsaturated fatty acids, in particular docosahexaenoic acid using marine microorganisms.

### Background of the invention

Polyunsaturated fatty acids (PUFAs) can be subdivided into three main groups, *i.e*. a group comprising ω-3 polyunsaturated fatty acids (n-3), a group comprising ω-6 polyunsaturated fatty acids (n-6) and a group comprising ω-9 polyunsaturated fatty acids (n-9). PUFAs are important components of the plasma membrane of cells, where they may be found in such forms as for example phospholipids. Alternatively, PUFAs can also be present in a cell as triglycerides. PUFAs are *inter alia* necessary for proper development, particularly in the developing infant brain and for tissue formation. PUFAs also serve as precursors to other molecules of importance in human beings and animals, including the prostacyclins, eicosanoids, leukotrienes and prostaglandins.

ω-3 Polyunsaturated fatty acids are of significant commercial interest. DHA for instance, an important PUFA in human breast milk, is important for infant development. Therefore, infant food manufacturers are interested in adding DHA into infant food products.

Furthermore, PUFAs have been recognised as important dietary compounds for preventing arteriosclerosis and coronary heart disease, for alleviating inflammatory conditions and for retarding the growth of tumor cells. These beneficial effects are a result both of ω-3 polyunsaturated fatty acids causing competitive inhibition of compounds produced from ω-6 polyunsaturated fatty acids, and from beneficial compounds produced directly from the ω-3 polyunsaturated fatty acids themselves. A well-known ω-3 polyunsaturated fatty acid is docosahexaenoic acid (C22:6 (n-3); DHA). The primary commercial source of this ω-3 polyunsaturated fatty acid is fish oil such as oil from menhaden, herring, tuna and sardines. The disadvantages of the use of fish oils as sources of DHA are *inter alia* problems associated with the recovery of fish oils, as well as the low and variable content of DHA in the fish oils. Furthermore, fish oils have an unpleasant odour and taste and are susceptible to contamination with environmental pollutants such as PCB's.

Besides in fish, DHA can also be found in marine microorganisms. In particular, various species of dinoflagellates are known to contain DHA. Methods of producing DHA by culturing dinoflagellates are known in the art. A dinoflagellate species preferably used in these methods is *Crypthecodinium cohnii* (see WO 91/11918 and WO 01/04338), because this species contains a fatty acid profile in which DHA is the only PUFA present in sufficient quantities.

WO 91/11918 and WO 01/04338 disclose a method of producing DHA, wherein *Crypthecodinium cohnii* is cultured with glucose or acetic acid/acetate, respectively, as carbon source. A disadvantage of both methods is that the overall volumetric productivity of DHA (r_{DHA}) is low. This productivity is particularly important in industrial cultivation processes for the production of large amounts of DHA by *C*. *cohnii.* A further disadvantage of the use of acetic acid/acetate as carbon source is corrosion of equipment leading to higher costs. Besides that, the use of acetic acid/acetate can lead to situations (for instance spilling of fluids) that are unsafe for production workers. A specific disadvantage of a method of producing DHA by *C. cohnii* with glucose as carbon source according to WO 91/11918 is that a stationary phase of *C. cohnii* has to be imposed to achieve satisfactory production of DHA in this process. This has as a consequence that the method is inefficient.

The problem to be solved according to the invention is to provide a process of producing polyunsaturated fatty acids from marine microorganisms which does not have the drawbacks mentioned above.

### Summary of the invention

It was found according to the invention that the above-mentioned disadvantages are absent in a process of producing DHA by culturing marine microorganisms, preferably dinoflagellates such as for instance *Crypthecodinium cohnii,* with a monoalcohol as carbon source. The use of this carbon source results on the one hand in r_{DHA}-values that are higher than the r_{DHA}-values reached when glucose or acetic acid/acetate are used as carbon sources and thus results in higher amounts of DHA produced by the marine microorganisms. In addition, the use of monoalcohols further reduces the costs. Firstly, because monoalcohols are relatively inexpensive substrates compared to acetic acid/acetate and glucose. Secondly, because the costs relating to the corrosive properties of acetic acid/acetate are avoided when monoalcohols are used as carbon source. Furthermore, the yield of biomass on ethanol is higher than on acetic acid, leading to a higher rate of DHA production and thus to lower production costs. In addition, monoalcohols are safer than acetic acid/acetate and the use of monoalcohols, contrary to the use of glucose, does not impose a stationary phase in order to achieve a satisfactory production of DHA.

### Description of the invention

### Definitions

### Polyunsaturated fatty acid

As used herein, the term "polyunsaturated fatty acid" (PUFA) refers to fatty acids, *i.e.* a class of compounds containing a long hydrocarbon chain (typically C16 to C28) and a terminal carboxyl group and having at least two alkenyl, *i.e.* double, bonds. The system commonly used to denote the structure of the polyunsaturated fatty acid uses the letter "C" accompanied by a number denoting the number of carbons in the hydrocarbon chain, followed by a colon and a number indicating the number of double bonds, *i.e.,* C20:5, eicosapentaenoic acid. Carbon atoms in the polyunsaturated fatty acid are numbered starting at the carboxy carbon.

The position of the double bonds can be indicated on the one hand by adding the capital delta (Δ) followed by a superscript number of the first carbon atom of the double bond, *i.e.* Δ⁹ means that there is a double bond between carbon atoms 9 and 10.

Alternatively, the position of a double bond can be denoted by counting from the methyl carbon atom at the distal end of the chain. This carbon atom is called the ω-carbon. For instance, ω-3 polyunsaturated fatty acids are understood to be polyunsaturated fatty acids that contain a double bond in the third position from the methyl group. The position of the double bond in the third position from the methyl group of ω-3 polyunsaturated fatty acids is also denoted as (n-3). This feature is located after the number of carbons in the hydrocarbon chain, the colon and the number indicating the number of double bonds, *i.e*. C22:6 (n-3) or C20:5 (n-3).

### Monoalcohol

An organic molecule, in particular an aliphatic molecule having up to 6, in particular up to 4 carbon atoms and comprising one hydroxyl group.

### Dinoflagellate

As used herein, the term "dinoflagellate" relates to microscopic, (usually) unicellular, flagellated, microorganisms, often photosynthetic protists that are commonly regarded as "algae" (Division Dinoflagellata). There are nearly 2000 known living species of dinoflagellates, classified in about 125 genera.

They are characterised by a transverse flagellum that encircles the body and a longitudinal flagellum oriented perpendicular to the transverse flagellum.

Dinoflagellates possess a unique nuclear structure at some stage of their life cycle - a dinokaryotic nucleus (as opposed to eukaryotic or prokaryotic) - in which the chromosomes are permanently condensed. In addition, the DNA is not associated with histones as in other eukaryotic cells. Dinoflagellates contain a lot of DNA, which explains the large size of the nucleus.

The dinoflagellate cell is surrounded by a series of membranes called the amphiesma. The cell wall of many dinoflagellates is divided into plates of cellulose (armor) within amphiesmal vesicles, known as a theca. These plates form a distinctive geometry/topology known as tabulation, which is the main means for classification.

Both heterotrophic, *i.e*. those who use organic materials, and autotrophic (photosynthetic) dinoflagellates are known. Some are both. Photosynthetic dinoflagellates utilise energy from sunlight and provide a food source for other organisms. The photosynthetic dinoflagellates are important primary producers in coastal waters. Approximately half of all species are heterotrophic, utilising organic materials for growth.

Most dinoflagellates go through moderately complex life cycles involving several steps, both sexual and asexual, motile and non-motile. The most common form of reproduction is asexual, where daughter cells are formed by simple mitosis and division of the cell. The daughter cells will be genetically identical to the original cell. Under some conditions sexual reproduction may occur. Some dinoflagellate species form cysts.

### Acids

The term "acids", in particular when referring to organic acids, implies organic compounds comprising an acidic group as well as organic compounds comprising an ionised form of this group such as for instance ionisable salts, in particular alkali metal, alkaline earth metal, ammonium and substituted ammonium salts, hydrolysable esters and the like.

### Polysaccharide-degrading enzyme

The term "polysaccharide degrading enzyme" as used herein relates to enzymes that can hydrolyse polysaccharides.

### Batch process

The term "batch process" as used herein pertains to a process of culturing marine microorganisms, wherein a certain volume of a culture medium is inoculated with the marine microorganisms. The marine microorganisms grow in the medium and are exposed to changing conditions, as nutrients are depleted and fermentation products may accumulate. During the process air or oxygen enriched air is lead through the medium and volatile products and/or CO₂ can leave the fermentation broth. Furthermore, alkali or acid is added to regulate the pH and anti-foaming agents may be added.

### Fed-batch process

The term "fed-batch process" as used herein concerns a process of culturing marine microorganisms, wherein a certain volume of a culture medium is inoculated with the marine microorganisms. One or more nutrients are added during the culturing process, but no removals are performed. During the process air or oxygen enriched air is lead through the medium and volatile products and/or CO2 can leave the fermentation broth. Furthermore, alkali or acid is added to regulate the pH and anti-foaming agents may be added.

### Continuous process

The term "continuous process" as used herein pertains to a process of culturing marine microorganisms under conditions in which additions to and removals from the culture medium take place during the entire duration of the process. In this way, nutrients and space are not exhausted during the period of culturing.

### Detailed description of the invention

In a first aspect the present invention concerns a process of producing a polyunsaturated fatty acid by a marine microorganism, the process comprising the steps of culturing the marine microorganism in the presence of at least one monoalcohol as carbon source in the culture medium, and recovering the polyunsaturated fatty acid from the marine microorganism or the culture medium thereof. Alternatively, the complete or disrupted biomass, *i.e.* marine microorganism, comprising the polyunsaturated fatty acid may be applied.

Polyunsaturated fatty acids that can be produced according to the process of the invention include fatty acids and their derivatives having from 16 to 28 carbon atoms and having at least two C-C double bonds. Preferably these double bonds are *cis* bonds, each subsequent double bond being located at the third C-C bond from the preceding one. These include, but are not limited to, ω-9 polyunsaturated fatty acids such as *inter alia* 6,9-octadecadienoic acid (C18:2 (n-9)); 8,11-eicosadienoic acid (C20:2 (n-9)); 5,8,11-eicosatrienoic acid (mead acid; C20:3 (n-9)).

Furthermore, these also include, but are not limited to, ω-6 polyunsaturated fatty acids such as *inter alia* 9,12-octadecadienoic acid (linoleic acid; C18:2 (n-6)); 6,9,12-octadecatrienoic acid (*gamma*-linolenic acid; C18:3 (n-6)); 8,11,14-eicosatrienoic acid (DGLA, 20:3); 5,8,11,14-eicosatetraenoic acid (arachidonic acid; C20:4 (n-6)); 7,10,13,16-docosatetraenoic acid (C22:4, (n-6)); 4,7,10,13,16 docosapentaenoic acid (C22:5, (n-6)); 4,7,10,13,16,19,22 octacosaheptaenoic acid (C28:7 (n-6)) and ω-3 polyunsaturated fatty acids such as *inter alia* 9,12,15-octadecatrienoic acid (*alpha*-linolenic acid; C18:3 (n-3)); 6,9,12,15, octadecatetraenoic acid (stearidonic acid; C18:4 (n-3)); 8,11,14,17 eicosatetraenoic acid (C20:4 (n-3)); 5,8,11,14,17-eicosapentaenoic acid (EPA; C20:5 (n-3)); 7,10,13,16,19 docosapentaenoic acid (C22:5 (n-3)); 4,7,10,13,16,19-docosahexaenoic acid (DHA, C22:6 (n-3)); 6,9,12,15,18,21-tetracosahexaenoic acid (C24:6 (n-3)); 4,7,10,13,16,19,22,25-octacosaoctaenoic acid (C28:8 (n-3)). ω-3 Polyunsaturated fatty acids, particularly DHA (C22:6 (n-3)), are preferably produced according to the process of the invention.

In the process according to the invention the selected marine organism is cultured in the presence of at least one monoalcohol as carbon source in the culture medium. This culture medium is preferably a liquid medium. The monoalcohol according to the invention can be methanol, ethanol, propanol, butanol, pentanol or hexanol, including isomers of propanol, butanol, pentanol and hexanol, with ethanol being the preferred monoalcohol. Preferably, the monoalcohol or mixture of at least two different monoalcohols according to the invention is the carbon source in the culture medium. Alternatively, the carbon source in the culture medium can be a mixture of at least one monoalcohol with at least one conventionally used carbon source selected from the group that includes, but is not limited to, carbohydrates such as glucose, galactose, fructose, xylose, saccharose, maltose, dextrins, soluble starch, fucose, glucosamine and dextran, glycerol, glycerophosphate, mannitol, and acids, oils and lipids comprising various precursors of polyunsaturated fatty acids, especially of DHA. When the carbon source in the culture medium is a mixture of carbon-containing compounds, the monoalcohol or mixture of monoalcohols is preferably the main carbon source in the culture medium, *i.e*. constitutes at least 50% w/w of the carbon-containing compounds.

During the fermentation the monoalcohol is consumed. In a fed-batch or a continuous process the monoalcohol is added to the culture medium in such a way that the concentration of the monoalcohol in the culture medium according to the process of the invention is higher than 0 and lower than 25 g/l, preferably higher than 0.1 and lower than 10 g/l and more preferably higher than 0.2 and lower than 5 g/l. In a preferred embodiment the minimum amount of monoalcohol in the culture medium is preferably close to zero - i.e. lower than 1 g/l - in order to avoid e.g. growth reduction due to alcohol toxicity. This can be achieved by controlling the monoalcohol concentration.

A typical fermentation run requires from about 100 to about 400 g ethanol/l over the course of a run. Preferably, the addition of ethanol is regulated in such a way that the medium comprises a sufficient amount of ethanol for the microorganism to grow well, whereas the amount is low enough to not cause ethanol toxicity. At the start of the fermentation preferably 0.2 - 1.5 g/l/h ethanol was added, more preferably 0.4 - 1.2 g/l/h and most preferably 0.7 - 1.0 g/l/h. During the complete fermentation on average 1 - 4 g/l/h ethanol was added, more preferably 1.5 - 3.5 g/l/h and most preferably 2 - 3 g/l/h.

The culture medium to be used according to the invention further comprises at least one nitrogen source selected from natural nitrogen sources such as *inter alia* peptone, yeast extract, malt extract, meat extract, casamino acid, corn steep liquor, soybean cake, and amino acids; organic nitrogen sources such as *inter alia* urea and inorganic nitrogen sources such as *inter alia* ammonium sulfate, ammonium chloride and ammonium nitrate or other nitrate salts. In the case yeast extracts are used (these extracts may also contribute a carbon source to the culture medium) the preferred concentration of these extracts in a fed-batch or continuous process is 1-25 g/l, more preferably 3-20 g/l, and particularly 5-15 g/l.

The culture medium further comprise phosphates such as *inter alia* sodium and potassium phosphates and potassium dihydrogen phosphate or phosphates present in the yeast extracts mentioned above.

Furthermore, the culture medium can comprise inorganic salts such as *inter alia* ammonium sulfate, sodium sulfate, magnesium sulfate, iron sulfate, copper sulfate, magnesium chloride and calcium chloride. Next to inorganic salts the culture medium can also comprise sulfate, such as sulfate present in sea salts.

Besides that, the culture medium according to the fed-batch or continuous process of invention can comprise vitamins and other micronutrients such as *inter alia* salts such as the above-mentioned sea salts in a concentration of 1-50 g/l, preferably 5-40 g/l, most preferably 10-30 g/l, amino acids, growth factors, biotin, vitamin B12 or thiamine (preferably present in the above-mentioned yeast extracts).

Preferably the microrganisms according to the invention are cultured during 50 - 400 h, preferably 100 - 300 h at 10 - 35 °C, preferably 15 - 30°C, most preferably 20 - 28°C, at a pH of 5 - 9, preferably 6 - 8 and most preferably 6.5 - 7 and an O₂-concentration that is higher than 5%, preferably higher than 15% and most preferably higher than 30%.

The polyunsaturated fatty acids produced according to the process of the invention can be recovered from the culture medium of the marine microorganisms or preferably from the marine microorganisms themselves. Recovering of polyunsaturated fatty acids from culture medium can be achieved by conventional methods known in the art such as solvent extraction. In a preferred embodiment of the invention the marine microorganism cells are first separated from the culture medium by conventional means such as *inter alia* filtration, flocculation or centrifugation. Next, optionally, the cells are washed with a suitable washing fluid such as water and then optionally dried. The cells are optionally disrupted by homogenisation or ultrasonication and the oil fraction of the microorganisms is extracted with a suitable solvent such as ether, hexane, isopropyl alcohol, other hydrocarbons, methanol, ethanol, chloroform, dichloromethane, petroleum ether or supercritical fluids or with a mixed solvent system such as a system of ethanol/diethyl ether or a system of chloroform/methanol/and optionally water. When the marine microorganisms are not dried before extraction, solvents compatible with water such as methanol, ethanol and isopropanol or suitable mixed solvent systems such as isopropanol/hexane are preferred. Thereafter the solvent is removed from the extracted oil fraction by evaporation or distillation techniques known in the art. The polyunsaturated fatty acids are present in neutral lipids such as *inter alia* triacyl glycerols, diacyl glycerols and monoacyl glycerols or polar lipids such as *inter alia* phosphatidylcholine, -ethanolamine, -serine and -inositol in the extracted oil fraction. These lipids can be separated by conventional techniques known in the art. These techniques include, but are not limited to, solvent separation, column chromatography (adsorption chromatography, ion-exchange chromatography), thin layer chromatography, gas-liquid chromatography. The polyunsaturated fatty acids can be separated from the polar lipids by hydrolyses and further concentration and separation of the resulting fatty acids or fatty acid esters by using conventional methods known is the art such as *inter alia* column chromatography.

The marine microorganism used in the process according to the invention can be selected from heterotrophic and phototrophic/heterotrophic marine organisms. These include, but are not limited to the genera, *Crypthecodinium sp., Traustrochytrium sp., Schizochytrium sp., Nitzschia sp., Chlorella sp., Tetraselmis sp. ,Brachiomonas sp., Neospongiococcum,* and *Phaeodactylum sp.*

These marine microorganisms according to the invention can be wild-type, mutant or recombinant strains or a mixture thereof. The mutant or recombinant strains encompass strains that are able to synthesise larger amounts of lipids, preferably lipids comprising polyunsaturated fatty acids as compared to wild-type strains utilising the same substrates. Furthermore, the mutant or recombinant strains can be strains unable to produce polysaccharides.

In a preferred embodiment the marine microorganism is a dinoflagellate. The dinoflagellate is preferably *Crypthecodinium cohnii*. More preferably, *inter alia* the *Crypthecodinium cohnii* strains ATCC 40750, ATCC 30541, ATCC 50298, ATCC 30557 and most preferably strain ATCC 30772 are used according to the process of the invention.

A further aspect of the invention relates to a process comprising two phases. In the first phase the marine microorganism is cultured in the presence of at least one monoalcohol as a carbon source in the culture medium. In the second phase the marine microorganism is cultured in the presence of at least one organic acid as a carbon source in the culture medium. In this embodiment the step of culturing the marine microorganisms with a monoalcohol serves as the biomass and lipid production phase and the step of culturing the marine microorganisms with an organic acid further improves the lipid accumulation. The organic acid to be used in such second phase may be an organic acid having 1-6, in particular 2-4 carbon atoms, such as *inter alia* acetic, glycolic, propionic, pyruvic, butyric, isobutyric, lactic, malic or succinic acid. A preferred organic acid is acetic acid. If desired a mixture of organic acids can be used. At a selected timepoint, the addition of monoalcohol (alcohol feed) is replaced by addition of the organic acid (acid feed). Preferably, at this timepoint the monoalcohol in the culture medium is completely utilised by the microorganism but, alternatively, the organic acid can also be added to the culture medium comprising the monoalcohol.

The concentration of the organic acid in the culture medium according to the fed-batch or continuous process of the invention is 0.5-20 g/l, preferably 2-8-15 g/l and more preferably 6-9 g/l.

The production of certain polysaccharides by the marine microorganisms is unfavourable for the production of polyunsaturated fatty acids by the marine microorganisms, because the production of such polysaccharides increases the viscosity of the culture medium. This decreases the oxygen transfer rate by strongly decreasing the oxygen transfer coefficient (*k*_{*1*}A). The drop in *k*_{*1*}A may especially be relevant during large-scale industrial cultivations, because large-scale bioreactors generally have a lower maximal *k*_{*1*}A than laboratory-scale bioreactors. The decrease of the oxygen transfer rate leads to lower cell densities of the marine microorganisms in the bioreactors and this leads to lower overall volumetric productivity of polyunsaturated fatty acids giving rise to higher production costs of these fatty acids which is economically unfavourable. Another problem associated with the polysaccharide production is that the yield of polyunsaturated fatty acids can decrease, because a part of the carbon source present in the culture medium is used for polysaccharide production. A further problem of the viscosity of the culture medium is the downstream processing of the marine microorganisms, because filtration of cells is difficult. Finally, due the high viscosity, the culture medium needs to be vigorously stirred to ensure oxygen transfer. This vigorous stirring requires energy input which is economically unfavourable and induces heat production which is also unfavourable, especially in large-scale cultivations.

The undesired effects of the production of polysaccharides may or may not significantly affect the production of polyunsaturated fatty acids from marine microorganisms.

In view of the above a further aspect of the invention pertains to a process as defined above further comprising the step of adding to the culture medium at least one polysaccharide-degrading enzyme. If desired a mixture of polysaccharide-degrading enzymes can be added to the medium. Preferably, the polysaccharide-degrading enzyme or the mixture of polysaccharide-degrading enzymes is added before or during the step of culturing the marine microorganism in the presence of at least one monoalcohol as carbon source in the culture medium.

Alternatively, mutant or recombinant marine microorganism strains producing low amounts of polysaccharides or strains that are unable to produce polysaccharides are preferred. These strains can be obtained *inter alia* by deleting at least one complete nucleotide sequence of a protein involved in the synthesis of polysaccharides, by deleting a part of said nucleotide sequence, said part being necessary and/or important for activity and/or functionality of the protein, or by substituting nucleotides of the nucleotide sequence that are necessary and/or important for the activity and/or functionality of the protein. Furthermore, these strains can be obtained by disrupting the nucleotide sequence. Disruption of the nucleotide sequence can occur *inter alia* by insertion of another nucleotide sequence into the nucleotide sequence. Next to that, these strains can be obtained by changing the orientation of a nucleotide sequence (inversion of orientation) leading to non-functionality of the protein. Other methods known in the art to construct and produce these strains will be apparent for a person skilled in the art and are enclosed herewith.

Yet another alternative are mutant or recombinant strains wherein one or more genes encoding polysaccharide-degrading enzymes are introduced and the polysaccharide degrading enzymes are expressed by the strains. The construction and production of these mutant or recombinant strains will be apparent for a person skilled in the art and are also a part of the present invention.

The polysaccharide-degrading enzyme according to the invention includes, but is not limited to, starch-degrading enzymes such as α-amylases (EC 3.2.1.1), 1,3-α-glucanase (EC), exo-1,4-α-D-glucanases such as amyloglucosidases (EC 3.2.1.3), (3-amylases (EC 3.2.1.2), α-glucosidases (EC 3.2.1.20), and other exo-amylases, and starch-debranching enzymes, such as isoamylase (EC 3.2.1.68), pullulanase (EC 3.2.1.41), and the like. Other polysaccharide-degrading enzymes according to the invention include cellulases such as exo-1,4-β-cellobiohydrolase (EC 3.2.1.91), exo-1,3-β-D-glucanase (EC 3.2.1.39), glucosidase (EC 3.2.1.21) and the like, and endoglucanases such as endo-1,3-β-glucanase (EC 3.2.1.6) and endo-1,4-β-glucanase (EC 3.2.1.4) and the like. Further polysaccharide degrading enzymes according to the invention include L-arabinases, such as endo-1,5-α-L-arabinase (EC 3.2.1.99), α-arabinosidases (EC 3.2.1.55) and the like, and galactanases such as endo-1,4-β-D-galactanase (EC 3.2.1.89), endo-1,3-β-D-galactanase (EC 3.2.1.90), galactosidase (EC 3.2.1.22), β-galactosidase (EC 3.2.1.23) and the like, and mannanases, such as endo-1,4-β-D-mannanase (EC 3.2.1.78), β-mannosidase (EC 3.2.1.25), α-mannosidase (EC 3.2.1.24) and the like, and xylanases, such as endo-1,4-β-xylanase (EC 3.2.1.8), β-D-xylosidase (EC 3.2.1.37), 1,3-β-D-xylanase and the like, and other enzymes such as α-L-fucosidase (EC 3.2.1.51), α-L-rhamnosidase (EC 3.2.1.40), levanase (EC 3.2.1.65), inulanase (EC 3.2.1.7) and the like. Alternatively, commercial preparations comprising at least one of the above or other polysaccharide-degrading enzymes such as for instance Glucanex (Novo Nordisk) can be used.

The polysaccharide-degrading enzyme may be used in the first step of the process of the invention, *i.e.* the biomass production step wherein monoalcohol is fed. If an additional culture step is carried out for lipid accumulation as described above, the polysaccharide-degrading enzyme may be used in this step as well. The polysaccharide-degrading enzyme may be added during the process or at a specific time point before or after one of the steps of the process.

In an embodiment the marine microorganism culture of the process according to the invention is performed as a batch process. For details see the examples section

In another embodiment the marine microorganism culture of the process according to the invention is performed as a fed-batch process. See for details also the examples section.

In another embodiment the marine microorganism culture of the process according to the invention is performed as a continuous process.

The biomass of the marine microorganisms or the disrupted biomass of the marine microorganisms, as well as the polyunsaturated fatty acids obtained by the process according to the invention can be added to various liquid and solid foods for men, fish and/or animals. Furthermore, they can be added as an additive for instance cosmetics and as raw materials for pharmaceuticals.

### Description of the figures

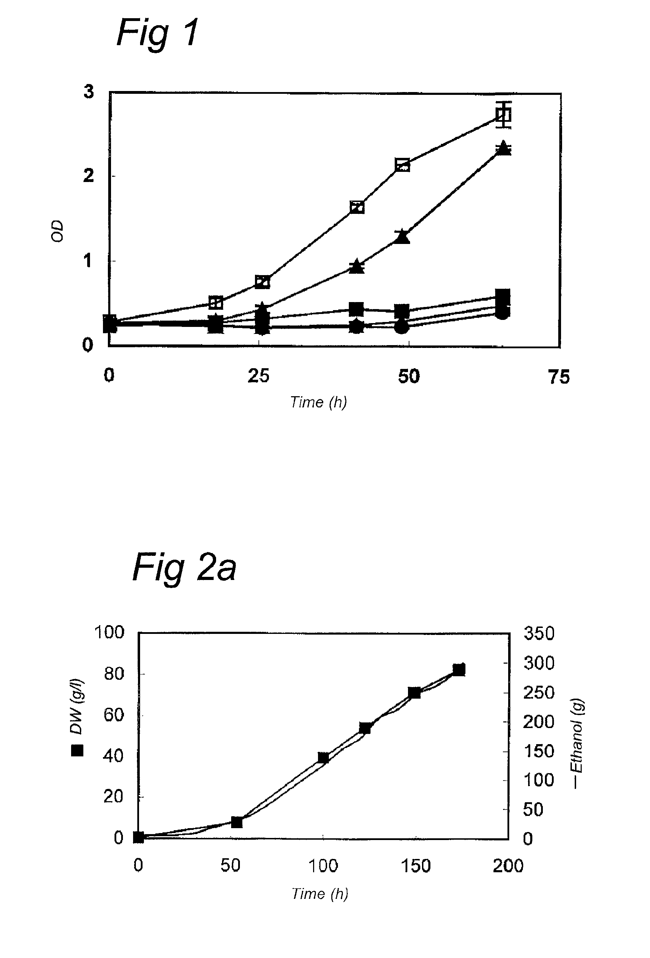

Figure 1 is a graphical illustration of the influence of the ethanol concentration on growth of *C*. *cohnii* in shaken flask batch cultivations. Medium: 2 g/L yeast extract, 25 g/L sea salt, 10% (v/v) inoculum and (■) 0, (□) 5, (▲) 10, (Δ)15 or (●) 25 g/L ethanol. The optical density (OD) was measured at 470 nm.

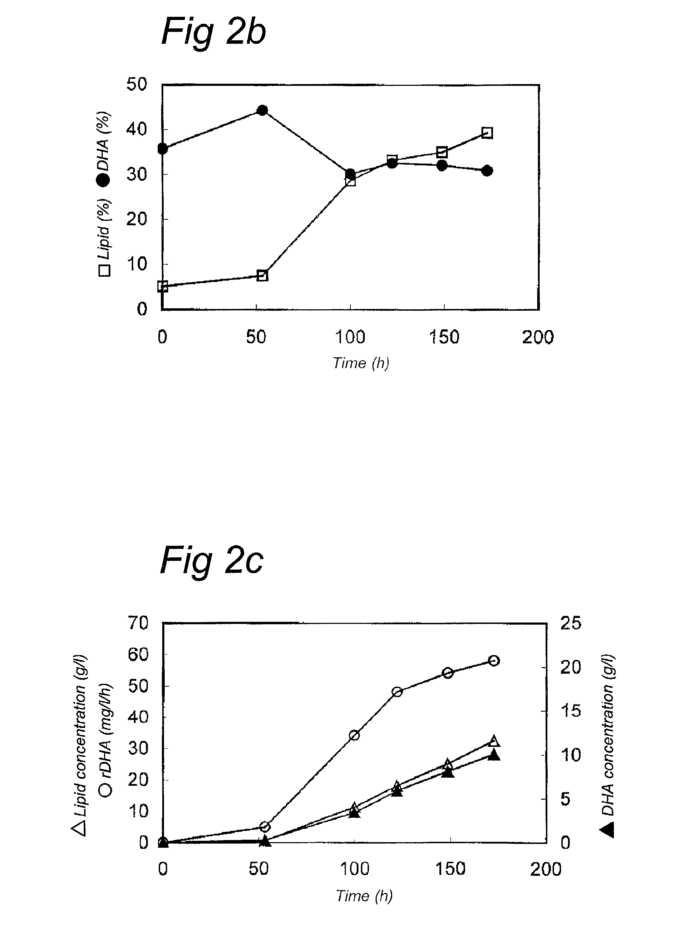

Figure 2 is a graph showing a fed-batch cultivation of C. *cohnii* with a feed consisting of pure ethanol. Initial medium: 10 g/L yeast extract, 25 g/L sea salt, 5.5 g/L ethanol, 0.5 g/l glucanex (Novo Nordisk) and 10% (v/v) inoculum. A: Biomass dry weight in g/l (DW; ■) and ethanol added in g (Ethanol). B: Lipid content of dry biomass in % (Lipid; □), DHA content of lipid in % (DHA; ●). C: Lipid concentration in g/l (Δ), overall volumetric productivity of DHA in mg/l.h (rDHA; ○) and DHA concentration in g/l (▲).

### Examples

### Inoculation of C. cohnii

*C. cohnii* strain ATCC 30772 obtained from the American Type Culture Collection is cultivated in a fermentor (Applicon) using ethanol as carbon source and synthesising DHA.

First, the inoculum is prepared from a standing culture of this strain using a pre-inoculation medium. The pre-inoculation medium contains (initially) glucose (9 g/l), yeast extract (2 g/l) and sea salts (25 g/l). Before use, the pH of the pre-inoculation medium is corrected to a pH in the range of 6.5 - 6.7 by the addition of HCl or NaOH and the medium is autoclaved at 121°C for 30 minutes (the glucose and yeast extract are sterilised separately to avoid caramelisation and precipitation of salts). The inoculum is prepared by adding 10% (v/v) of the standing culture to a 300 ml flask containing 50 ml of the pre-inoculation medium. The flask is incubated at 27°C while shaking at 100 rpm for 3 days. 10 ml of the incubated mixture is then added to a 500 ml flask containing 100 ml medium with glucose (25 g/l), yeast extract (5.5 g/l) and sea salt (25 g/l). This flask is then incubated while shaking at 100 rpm for 3 days after which the content is used as inoculum for the fermentor.

### Batch growth of C. cohnii with ethanol as carbon source

In shaken flask cultivations *C. cohnii* is able to utilise ethanol at concentrations between 0.5 and 15 g/l with the preferred concentrations being between 5 and 10 g/l (see figure 1). An ethanol concentration of 10 g/l showed inhibition of growth as compared to 5 g/l. Concentrations above 15 g/l, resulted in no significant growth during 3 days of incubation. No significant growth also occurred without ethanol. The yeast extract present in the growth medium is provided, preliminarily, as a source of nitrogen, essential vitamins and growth factors. The sea salts provide the so called "mineral spore elements" and are needed as nutrients and for osmo-protection of the *C. cohnii* cells.

### Fed-batch growth of C. cohnii with ethanol as carbon source

Fed-batch cultivation of *C. cohnii* was performed at 27°C in 2-1 laboratory bioreactors (Applikon Dependable Instruments, Schiedam, NL). The dissolved-oxygen tension (DOT), measured by an oxygen-electrode, was kept above 30% of air saturation by automatically controlling the stirrer speed (range: 200-1250 rpm) and by flushing (1 l/min) with filter-sterilised air. The stirrer speed was programmed only to increase and not to decrease. Foam production in the cultivations was repressed by addition of 3-6 droplets of MAZU DF 8005 antifoam (BASF, Cheadle, UK).

In fed-batch cultivations with ethanol as carbon source, the initial medium (11) contained 10 g/l yeast extract; 25 g/l sea salt, 5.5 g/l ethanol, 0.5 g/l glucanex and a 10% (v/v) inoculum. During the first 24-29 h of cultivation no ethanol was fed. A DOT spike raising above 60% of air saturation automatically started the pump. During the cultivation the ethanol concentration in the medium was kept below toxic values by stopping the ethanol feed whenever the dissolved oxygen tension rose above 35 %. The pH was kept at 6.5 ± 0.1 by automated addition of 2M HCl or 0.5 M NaOH. Under these conditions *C. cohnii* strain 30772 grows readily.

### A control mechanisms for the feeding of ethanol into fed-batch cultivations of C. cohnii

A fed-batch cultivation strategy of *C. cohnii* was developed with ethanol as carbon source. As *C*. *cohnii* produces a viscous polysaccharide during growth a commercial preparation comprising polysaccharide degrading enzymens (Glucanex, 0.5 g/l) was added to the media to decrease culture viscosity thereby facilitating aeration.

Initial cultivations showed that the dissolved oxygen tension (DOT) of the cultivation broth is a possible on-line variable for input of an automatic control of the ethanol feed. The depletion of ethanol in cultures became apparent by a quick rise of the DOT from 30% to >60% air saturation. This quick DOT rise was used as a switch to automatically start the ethanol feed *e.g*. after the batch phase.

Initial cultivations showed that when the ethanol was pumped too quickly into the medium over time the DOT slowly started to rise indicative for growth inhibition. High ethanol concentrations slow down growth (see figure 1), however, when the feed was stopped directly after the first signs of overdosage the effect was minimal and cells were quickly able to consume the excessive ethanol. The slow increase of the DOT during ethanol feeding was used in the automatic control of the ethanol feed. Once the DOT raised above 35 % of air saturation the ethanol feed was stopped and not switched on again until a quick DOT rise occurred. Importantly, after a quick DOT rise the pump was not switched off for 3 hours in order to allow the DOT to drop below 35 % again. An advantage of this method of cultivation is that it maintains the ethanol concentration between certain limits so that it is optimal for the process. The system of adding ethanol in response to a change in DOT is a relevant way of providing ethanol as it is needed to replenish ethanol used by the *C. cohnii* cells. The addition of ethanol in this way allows optimum growth of the *C. cohii* cells, especially the *C. cohnii* cells of the strain 30772, a high final biomass (consisting of cells of *C. cohnii* strain 30772), a high content of oil in the cells and a high proportion of docosahexaenoic acid within the oil.

Alternative to the described feeding process a more or less fixed, non-toxic, ethanol concentration may be maintained throughout the process. This can be achieved by using an ethanol sensor and use it's signal as input for the automatic control of the ethanol doses. For industrial applications a programmed feed of an optimal feeding strategy would be most practical combined with a toxicity control like the DOT signal, an ethanol sensor, or measurement of CO₂ or O₂ in the off-gas.

### Ethanol-grown fed-batch cultivation of C. cohnii

Prevention of ethanol starvation and toxic ethanol concentrations can be automatically controlled. The ethanol demand of the cells is dependent on cell concentration and the growth phase. The development of the feeding strategy was an iterative process; knowledge of previously run cultivations was implemented into the feeding patterns of following cultivations. By adding ethanol over a period of time, a considerable amount of ethanol can be provided as a carbon source for the *C. cohnii* strain 30772 while, at any particular moment in time, the concentration of ethanol is sufficient low as not to be detrimental to growth of the *C. cohnii* strain 30772.

An ethanol-grown fed-batch cultivation of *C. cohnii* yielded a biomass concentration of 82 g/l dry weight in 173 h (see figure 2). In this period approximately 290 g ethanol was added to the culture. The estimated specific growth rate over the first 53 h of incubation was 0.051 h⁻¹. The growth was linear between 53 and 173 h cultivation. The total lipid content of the dry cells increased most significantly between 53 and 100 h of cultivation and was maximal after 173 h, namely 39 %. The DHA content of the lipid varied between 31 and 44 %, but remained constant at 32±1 % after 100 h. Overall volumetric productivity of DHA (r_{DHA}) showed a maximum of 58 mg/l.h at 173 h. Values for dry weight, lipid and DHA concentration at this time point of cultivation were 82, 32 and 10 g/l, respectively.

After 173 h of cultivation cells and the growth medium are removed from the fermentor and the biomass (*C. cohnii* cells) is separated from the growth medium by centrifugation. Biomass concentration and the oil content of the biomass is determined using known methods (see De Swaaf *et al.* Appl. Microbiol. Biotechnol. (2001) 57, 395-400). The biomass itself, in the form of cells and/or disrupted cells, as well as the oil thereof contains docosahexaenoic acid and is useful as food, food additive, for pharmaceutical applications, as cosmetic ingredient or as feed ingredient. Purified docosahexaenoic acid and also esters of docosohexaenoic acid with simple alcohols may be obtained from the oil by methods known in the art. The purified docosahexaenoic acid and esters thereof can also be used as food, food additive, for pharmaceutical applications, as cosmetic ingredient or as feed ingredient.

Comparison of ethanol- and acetic acid grown fed-batch cultivations showed that after 173 h biomass was produced faster with ethanol, whereas the lipid content of the dry biomass was higher with acetic acid as carbon source. Nevertheless, significant higher amounts of DHA (g/l) and r_{DHA}-values are reached in ethanol-grown cultures than in acetic-acid grown cultures (see table 1).

**Table 1.**

| Comparison of representative, ethanol and acetic acid fed-batch cultures of *C. cohnii* strain 30772. Selected parameters are shown for time point 173 h. | | |
|---|---|---|
| | Ethanol feed | Acetic acid feed |
| Time (h) | 173 | 173 |
| Biomass (g/l) | 82 | 40 |
| Lipid content (% w/w) | 39 | 53 |
| Lipid concentration (g/l) | 32 | 21 |
| DHA in lipid (% w/w) | 31 | 29 |
| DHA concentration (g/l) | 10 | 6 |
| r_{DHA} (mg/l.h) | 58 | 36 |

## Claims

1. A process of producing a polyunsaturated fatty acid by using a marine microorganism, the process comprising the steps of:
a) culturing the marine microorganism in the presence of at least one monoalcohol as a carbon source in the culture medium,
b) recovering the polyunsaturated fatty acid from the marine microorganism or the culture medium.

2. A process according to claim 1, wherein the monoalcohol is selected from the group consisting of methanol, ethanol, propanols, butanols, pentanols and hexanols.

3. A process according to claim 1 or 2, wherein the concentration of the monoalcohol in the culture medium is higher than 0 and lower than 25 g/l.

4. A process according to any of the claims 1-3, wherein the polyunsaturated fatty acid is docosahexaenoic acid.

5. A process according to any of the claims 1-4, wherein the marine microorganism is a dinoflagellate.

6. A process according to claim 5, wherein the dinoflagellate is of the genus *Crypthecodinium*.

7. A process according to claim 6, wherein the dinoflagellate is *Crypthecodinium cohnii*.

8. A process according to any of the claims 1-7, further comprising after step a) and before step b) the step of culturing the marine microorganism in the presence of at least one organic acid as a carbon source in the culture medium.

9. A process according to claim 8, wherein the organic acid is selected from the group consisting of acetic acid, glycolic acid, propionic acid, pyruvic acid, butyric acid, isobutyric acid, lactic acid, malic acid and succinic acid.

10. A process according to claim 8 or 9, wherein the concentration of the organic acid in the culture medium is 0.5-20 g/l.

11. A process according to any of the claims 1-10, further comprising adding to the culture medium at least one polysaccharide-degrading enzyme.
